# EUROPÄISCHE PATENTSCHRIFT

(11) **EP 0 766 972 B1**
(45) Veröffentlichungstag und Bekanntmachung des Hinweises auf die Patenterteilung: **13.02.2002**
(21) Anmeldenummer: 96111262.0
(22) Anmeldetag: 12.07.1996
(51) Int. Cl.: A61L 29/00, A61L 33/00

(54) **Medizinische Arbeitsmittel**
Medical instrument
Instrument médical

(30) Priorität: 08.09.1995 DE 19533245
(43) Veröffentlichungstag der Anmeldung: 09.04.1997
(73) Patentinhaber: REHAU AG + Co, 95111 Rehau (DE)
(72) Erfinder: Kühlein, Georg, 95111 Rehau (DE)

(56) Entgegenhaltungen:
- DE-A- 3 730 797
- US-A- 5 356 668

## Beschreibung

Die Erfindung betrifft medizinische Arbeitsmittel wie Katheter, Schlauche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen.

Aus der DE-A 19 30 136 ist ein beschichteter Katheter bekannt, welcher als Basis aus einem Schlauch aus Gummi oder einem Elastomeren besteht. Dieser Katheterschlauch ist an der Außen- und/oder Innenwand mit einem hydrophilen Polyacrylat bzw. -Methacrylat, insbesondere mit einem Hydroxialkyl- oder einem Hydroxialkoxi-Alkyl-Acrylat bzw. -Methacrylat mit jeweils niederen Alkylresten beschichtet.

Weiterhin ist es bekannt, derartige medizinische Arbeitsmittel mit einer Beschichtung aus Heparin zu versehen. Diese Heparin-Beschichtungsverfahren arbeiten über APTES- und TDMAC-Brücken. Mit dieser Heparin-Beschichtung wird das Anhaften von Blutplättchen an der Oberfläche des medizinischen Arbeitsmittels verhindert und gleichzeitig die intrinsische Koagulation desaktiviert. Ausführungen zum Beschichtungsverfahren mit Heparin über TDMAC-Brücken finden sich bei G.A. Grode, R.D. Falb, J.P. Crowley in J. Biomed. Mater. Res. Symp., 3.77 (1972). Entsprechende Hinweise zur Beschichtung mit Heparin über APTES-Brücken finden sich bei R.L. Merker, L.J. Elyasch, S.W. Mayhew, J.Y.C. Wang in Proc. Artif. Heart Conf., 1969, Seite 29.

Alle diese Verfahren haben den prinzipiellen Nachteil, daß sie technisch aufwendig und sehr arbeitsintensiv sind. So läuft eine Heparinisierung bei beiden genannten Verfahren in mehreren zusätzlichen Arbeits- und Beschichtungszyklen ab.

Ein weiterer wesentlicher Nachteil dieser bekannten Verfahren ist, daß die auf diese Weise hergestellten medizinischen Halbzeuge sterilisiert werden müssen, weil die Beschichtung mit Heparin mikrobiell abgebaut werden kann. Da nach der Konfektion der Halbzeuge zu Fertigprodukten eine Gesamtsterilisation erforderlich ist, ist hier als weiterer Nachteil das erhebliche Risiko einer Mehrfachsterilisation zu nennen.

Neben der Verwendung von Heparin ist aus der DE 31 53 664 C2 die Verwendung von Polysiloxan-Polyurethan-Blockcopolymeren bekannt, um die Oberflächeneigenschaften von mit Blut in Berührung stehenden Oberflächen von Einrichtung biomedizinischer Geräte zu modifizieren. Diese Blockcopolymere haben selbst schlechte strukturelle Merkmale aufgrund des hohen Gehaltes an Polysiloxan. Außerdem handelt es sich um teure Rohstoffe und aufwendige Herstellungs- bzw. Beschichtungsverfahren.

Weiter ist aus der DE 19 44 969 A1 die Verwendung u.a. von Polyurethanen (Urethan-Siloxan-Polymeren) bekannt, welche zusammen mit einem Polysiloxan Blockmischpolymere bilden. Diese Blockmischpolymere zeichnen sich durch hohe mechanische Stabilität aus und können in Filme oder Platten gegossen werden. Sie besitzen außerdem eine hohe Blutverträglichkeit und eignen sich daher für die Oberflächen von Formteilen, die mit Blut in Berührung kommen. Die Verwendungsfähigkeit für Beschichtungen ist allerdings begrenzt bzw. nicht gegeben.

Aus der EP 0 334 062 A2 ist die Verwendung von Polycaprolactom-Polymeren bei medizinischen Artikeln bekannt, deren Oberfläche eine blutverträgliche Beschichtung damit aufweist. Mit diesen Beschichtungen wird beispielsweise das Entstehen von Blutgerinnseln verhindert. Ferner ist aus der EP 0 261 470 A1 eine vergleichbare Anwendung von Polycaprolactom-Polymeren und aus der EP 0 068 385 B1 eine Verwendung von Polyurethan-Silikon-Polymeren für unmittelbar mit Blut in Kontakt gebrachte Formteile bekannt.

Obwohl diese Vorveröffentlichungen die antithrombotische Behandlung von medizinischen Artikeln beschreiben, ist zum einen die optimale Blutkompatibilität nicht gegeben und zum anderen handelt es sich um arbeitsaufwendige und damit kostenintensive Verfahrensweisen.

Hier setzt die Erfindung ein, die es sich zur Aufgabe gestellt hat, die zum Stand der Technik genannten Nachteile zu vermeiden und eine Methode zur Erzielung der optimalen Blutkompatibilität bei medizinischen Halbzeugen aufzuzeigen, bei der über das medizinische Arbeitsmittel weder das biologische System negativen Auswirkungen ausgesetzt ist noch das Material, aus dem das medizinische Arbeitsmittel gefertigt ist, durch Einwirkung des biologischen Systems so weit geschädigt werden kann, daß es die vorgesehene Funktion nicht mehr erfüllt. Erfindungsgemäß wird dazu vorgeschlagen, daß in das Ausgangspolymere vor der eigentlichen Formgebung eine definierte Menge eines Copolymeren aus Hexafluor-Propylen und Vinylidenfluorid mit einem mittleren Molekulargewicht von wenigstens 70.000 eingemischt ist.

Die Erfindung geht von der Überlegung aus, dem Ausgangspolymeren Additive zuzusetzen, die den Werkstoff von der Blutkompatibilität her mit seinem relativen Gerinnungsparameter dem Quotienten 1,0 annähern.

Dieses Vorhaben ist durch die erfindungsgemäße Zugabe von Hexafluor-Propylen-Vinylidenfluorid-Copolymeren gelungen. So hat es sich bei der Verwendung von weichmacherhaltigem Polyvinylchlorid als Ausgangsmaterial für die Herstellung medizinischer Arbeitsmittel als vorteilhaft herausgestellt, daß die heute für solche Anwendungszwecke üblichen PVC-Rezepturen auf Basis Zink und Kalzium erfindungsgemäß durch die genannten Copolymere ergänzt werden. Hierbei hat sich als zweckmäßig eine Dosierungsrate von 0,1 bis 2,0 Gewichtsprozent erwiesen.

Bei der Verwendung anderer Polymere zur Herstellung der medizinischen Arbeitsmittel können die erfindungsgemäßen Copolymere die eingesetzten Arbeitshilfen wie Gleitmittel und dergleichen ergänzen oder ersetzen.

Die mit den erfindungsgemäßen Copolymeren ausgestatteten medizinischen Arbeitsmittel verhindern die Blutplättchen-Aggregation und desaktivieren die intrinsische Koagulation. Eine Vorsterilisierung der Halbzeuge ist nicht mehr erforderlich, da die erfindungsgemäßen Copolymere entgegen der bisher üblichen Heparinbeschichtung nicht mehr mikrobiell abgebaut werden können.

Die nachfolgenden Beispiele zeigen die Verwendungsmöglichkeit der erfindungsgemäßen Copolymere als Zuschlagstoffe für polymere Materialien zur Herstellung medizinischer Arbeitsmittel.

### Beispiel 1:

| Weich-PVC-Rezeptur | |
|---|---|
| 100 | Teile S-PVC |
| 10-90 | Teile organischen Weichmacher |
| 1-10 | Teile epoxidierte Öle |
| 0,1-1,0 | Teile Metallseifen |
| 0,1-0,6 | Teile Gleitmittel |
| 0,1-2 | Teile Hexafluor-Propylen-Vinylidenfluorid-Copolymer |

### Beispiel 2:

| Polyurethan-Rezeptur | |
|---|---|
| 100 | Teile Polyurethan |
| 0,1-1,5 | Teile Hexafluor-Propylen-Vinylidenfluorid-Copolymer |

### Beispiel 3:

| Polyethylen-Rezeptur | |
|---|---|
| 100 | Teile Polyethylen |
| 0,1-1,7 | Teile Hexafluor-Propylen-Vinylidenfluorid-Copolymer |

### Beispiel 4:

| Polypropylen-Rezeptur | |
|---|---|
| 100 | Teile Polypropylen |
| 1,0-3 | Teile Metallseifen |
| 0,1-2 | Teile Hexafluor-Propylen-Vinylidenfluorid-Copolymer |

## Patentansprüche

1. Medizinische Arbeitsmittel wie Katheter, Schläuche, Behälter, Formteile aus polymeren Materialien, wobei diese Arbeitsmittel eine besondere Ausstattung zur Erhöhung der Blutkompatibilität bei der Berührung mit Blut oder blutähnlichen Flüssigkeiten aufweisen, **dadurch gekennzeichnet, daß** in das Ausgangspolymere vor der eigentlichen Formgebung eine definierte Menge eines Copolymers aus Hexafluor-Propylen- und Vinylidenfluorid mit einem mittleren Molekulargewicht von wenigstens 70.000 eingemischt ist.

2. Medizinische Arbeitsmittel nach Anspruch 1, **dadurch gekennzeichnet, daß** die Menge der Hexafluor-Propylen-Vinylidenfluorid-Copolymere zwischen 0,1 und 2,0 Gewichtsprozent liegt.

## Claims

1. Medical equipment such as catheters, hoses, vessels, mouldings made of polymeric materials, in which this equipment is specially furnished to increase the blood compatibility on contact with blood or liquids similar to blood, **characterised by**: a defined quantity of a co-polymer consisting of hexafluoropropylene and vinylidene fluoride with an average molecular weight of at least 70,000 is added to the initial polymers before the actual moulding.

2. Medical equipment as described in Claim 1, **characterised by**: the quantity of hexafluoropropylene/vinylidene fluoride co-polymer lies between 0.1 and 2.0 percent by weight.

## Revendications

1. Instruments médicaux tels que cathéters, tuyaux, récipients, raccords en matériaux polymères présentant une composition particulière en vue d'améliorer leur compatibilité avec le sang lors du contact avec le sang ou des liquides similaires au sang, **caractérisés en ce qu'**avant le formage proprement dit, une quantité définie de copolymère constitué d'hexafluoropropylène et de fluorure de vinylidène d'un poids molaire minimum de 70.000 est mélangée au polymère de base.

2. Instruments médicaux selon la revendication 1, **caractérisés en ce que** la quantité de copolymère d'hexafluoropropylène - fluorure de vinylidène se situe entre 0,1 et 2,0 pour cent en poids.
